(19) Europäisches Patentamt
European Patent Office
Office européen des brevets

(11) Publication number: **0 209 980**
**B1**

(12) # EUROPEAN PATENT SPECIFICATION

(45) Date of publication of patent specification: **11.01.89**

(51) Int. Cl.[4]: **C 07 C 1/04**, B 01 J 23/76, B 01 J 23/78, B 01 J 29/24, B 01 J 29/34

(21) Application number: **86304542.3**

(22) Date of filing: **12.06.86**

(54) **Syngas conversion.**

(30) Priority: **15.06.85 GB 8515222**

(43) Date of publication of application: **28.01.87 Bulletin 87/05**

(45) Publication of the grant of the patent: **11.01.89 Bulletin 89/02**

(84) Designated contracting states: **BE DE FR GB IT NL**

(56) References cited:
DE-A- 2 750 007
DE-A- 3 316 320
DE-B- 2 739 466
GB-A- 1 458 247
US-A- 4 374 819
US-A- 4 399 234

(73) Proprietor: **The British Petroleum Company p.l.c.**
**Britannic House Moor Lane**
**London EC2Y 9BU (GB)**

(72) Inventor: **Mcateer, Colin Hugh**
**British Petroleum Company p.l.c. Chertsey Road**
**Sunbury-on-Thames Middlesex, TW16 7LN (GB)**
Inventor: **Nay, Barry**
**British Petroleum Company p.l.c. Chertsey Road**
**Sunbury-on-Thames Middlesex, TW16 7LN (GB)**

(74) Representative: **Fawcett, Richard Fennelly et al**
**BP INTERNATIONAL LIMITED Patents Division Chertsey Road**
**Sunbury-on-Thames Middlesex, TW16 7LN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 209 980 B1

Jouve, 18, rue St-Denis, 75001 Paris, France

**Description**

The present invention relates to the use of a novel catalyst in the conversion of gaseous mixtures comprising principally carbon monoxide and hydrogen (synthesis gas) into hydrocarbons of carbon number greater than one, in particular into aliphatic hydrocarbons in the gasoline boiling range.

The conversion of synthesis gas into hydrocarbons by the Fischer-Tropsch process has been known for many years but the process has only achieved commercial significance in countries such as South Africa where unique economic factors prevail. The growing importance of alternative energy sources such as coal and natural gas has focussed renewed interest in the Fischer-Tropsch process as one of the more attractive direct and environmentally acceptable routes to high quality transportation fuels.

Many metals, for example cobalt, nickel, iron, molybdenum, tungsten, thorium, ruthenium, rhenium and platinum are known to be catalytically active, either alone or in combination, in the conversion of synthesis gas into hydrocarbons and oxygenated derivatives thereof. Of the aforesaid metals, cobalt, nickel and iron have been studied most extensively. Generally, the metals are used in combination with a support material, of which the most common are alumina, silica and carbon.

The use of cobalt as a catalytically active metal in combination with a support has been described in, for example, EP-A-127 220, EP-A-142 887, GB-A-2 146 350, GB-A-2 130 113 and GB-A-2 125 062. EP-A-127 220, for example, discloses the use of a catalyst comprising (i) 3-60 pbw cobalt, (ii) 0.1-100 pbw zirconium, titanium, ruthenium or chromium per 100 pbw silica, alumina or silica-alumina, (iii) the catalyst having been prepared by kneading and/or impregnation.

Rare earth metals have also been used in a promotional capacity as components of syngas conversion catalysts. Thus, for example, J5 9179154A describes a syngas conversion catalyst obtained by depositing a catalytic iron group metal, preferably cobalt or iron, with manganese oxide, a platinum group metal, preferably ruthenium, rhodium, palladium, platinum or iridium, and at least one of alkali metal oxide, preferably potassium, sodium, lithium, cesium or rubidium or an alkaline earth metal oxide, for example magnesium, calcium, barium or strontium, or a rare earth metal oxide, preferably lanthanum, cerium, praesodymium or samarium, on a catalyst carrier consisting of silica and/or alumina.

We have now found that cobalt in combination with ceria and optionally also lanthana, optionally promoted by an alkali metal, catalyses the conversion of syngas into higher ($C_{3+}$) hydrocarbons.

Accordingly, the present invention provides a process for the production of hydrocarbons having a carbon number greater than one by contacting the synthesis gas at elevated temperature and atmospheric or superatmospheric pressure with a solid catalyst characterised in that the catalyst has a composition represented by the formula

$$Co_a \cdot A_b \cdot La_c \cdot CeO_x \qquad (I)$$

wherein

A is an alkali metal

a is greater than zero and up to 25 % w/w,

b is in the range from zero to 5 % w/w,

c is in the range from zero to 15 % w/w,

x is a number such that the valence requirements of the other elements for oxygen is satisfied, and the remainder of the composition, subject to the requirement for x, is cerium,

the percentages w/w being based on the total weight of the composition.

In the formula (I) A is an alkali metal, which largely for reasons of availability and cost, is preferably either sodium or potassium. Preferably the amount (b) of alkali metal is less than 2 % w/w, even more preferably it is zero. The value of a in the formula (I) is suitably less than 5 % w/w, preferably less than 1 % w/w. The value of c in the formula (I) is preferably greater than zero and preferably less than 10 % w/w.

Cerium may be present in the composition either alone or admixed with lanthanum or lanthanum and other lanthanide rare earth metal(s) oxides, such as for example those commonly found in commercially available technical grade ceria.

The catalyst having the formula (I) may be prepared by a variety of processes, for example by impregnation, by precipitation, by coprecipitation, by cogelation or by vapour deposition, or by any other process. One method of preparing the catalyst of formula (I) involves impregnating a ceria support with a solution of a soluble cobalt compound. The solution may be an aqueous solution or a solution in an organic solvent of a suitable compound. Suitable compounds include cobalt complexes, for example cobalt (II) acetylacetonate and cobalt (III) acetylacetonate, and cobalt salts such as a nitrate or a halide. In those catalysts incorporating an alkali metal, this may either be impregnated on to the rare earth metal oxide support simultaneously with the cobalt or may be added at a later stage in the preparation.

Alternatively, the cobalt may be precipitated on to the rare earth metal oxide support from a solution of a soluble compound thereof by treatment with a suitable precipitant, suitably by

(A) bringing together at a temperature below 100 °C ceria, a solution of a soluble compound of cobalt and a precipitant under conditions whereby cobalt is precipitated in the form of a heat decomposable compound, and

(B) recovering the mixture of the ceria and the precipitated cobalt compound obtained in step (A).

The amounts of the reagents employed should be such as to satisfy the stoichiometric relationships in the formula (I). In step (B) of the process the precipitate obtained in step (A) is recovered. This may suitably be accomplished by filtration but other methods for separating solids from liquids, for example centrifugation, may be employed. After recovery it is preferred to wash the precipitate, suitably with water, so as to remove unwanted residual soluble matter. It is also preferred to dry the precipitate, suitably at an elevated temperature below 200 °C, for example about 150 °C.

In a modification of the precipitation method hereinbefore described, a cerium compound or compounds convertible to ceria may be coprecipitated together with a cobalt compound.

For those catalysts containing lanthanum, the lanthanum may be incorporated at any stage in the preparative procedure.

Irrespective of whether the composition is prepared by impregnation, precipitation or coprecipitation or by any other method, it is preferred to carry out one or more additional steps before the composition is used as a catalyst. Thus it is preferred to roast the composition, suitably by heating it in, for example, a stream of gas such as nitrogen or air at a temperature suitably in the range from 250 to 600 °C. It is also preferred to reductively activate the composition, suitably by contact at elevated temperature with a reducing gas, for example hydrogen, which may be diluted with nitrogen. Typically, the conditions employed during the reductive activation step may suitably be a pressure in the range from 1 to 100 bar and a temperature in the range from 150 to 500 °C for a period of up to 24 hours or longer. Whilst it is preferred to effect the reductive activation step as a discrete step prior to use as a catalyst for the conversion of synthesis gas, it may be incorporated into the synthesis gas conversion process.

As is well known in the art synthesis gas principally comprises carbon monoxide and hydrogen and possibly also minor amounts of carbon dioxide, nitrogen and other inert gases depending upon its origin and degree of purity. Methods of preparing synthesis gas are established in the art and usually involve the partial oxidation of a carbonaceous substance, e. g. coal. Alternatively, synthesis gas may be prepared, for example by the catalytic steam reforming of methane. For the purpose of the present invention the carbon monoxide to hydrogen ratio may suitably be in the range from 2 : 1 to 1 : 6. Whilst the ratio of the carbon monoxide to hydrogen in the synthesis gas produced by the aforesaid processes may differ from these ranges, it may be altered appropriately by the addition of either carbon monoxide or hydrogen, or may be adjusted by the so-called shift reaction well known to those skilled in the art.

The elevated temperature may suitably be in the range from 190 to 400 °C, preferably from 200 to 350 °C. The pressure may suitably be in the range from 0 to 100 bar, preferably from 10 to 50 bar. The GHSV for continuous operation may suitably be in the range from 100 to 5 000 $h^{-1}$.

The process may be carried out batchwise or continuously in a fixed bed, fluidised bed or slurry phase reactor.

In a modification of the process of the present invention the catalyst of formula (I) may incorporate a suitable porometallotectosilicate. The porometallotectosilicate may suitably be an aluminosilicate zeolite, preferably an aluminosilicate zeolite having a high (that is greater than 10 : 1) silica to alumina ratio. Suitable aluminosilicate zeolites include, but are by no means restricted to, zeolite ZSM-5.

In a further modification, the process of the invention may include a further step in which the product or at least a portion thereof, obtained by contacting synthesis gas with the catalyst of formula (I) is up-graded by, for example, oligomerisation of lower olefins present therein to higher hydrocarbons in the manner described in, for example, US-A-4 544 792, US-A-4 520 215 and US-A-4 504 693 ; hydrocracking in the manner described in, for example GB-A-2 146 350 ; cracking and isomerisation of heavy by-products in the manner described in, for example, US-A-4 423 265 and up-grading in the manner described in, for example AU-A-8 321 809 and GB-A-2 021 145.

The invention will now be further illustrated by the following Examples. In the Examples CO conversion is defined as moles of CO used/moles of CO fed x 100 and carbon selectivity as moles of CO attributed to a particular product/moles of CO converted x 100.

A. Catalyst preparation

Cobalt/ceria catalysts were made by impregnating dry powdered ceria with a solution containing a cobalt compound. Cobalt loadings of 0.2, 0.3 and 0.5 % w/w were tested.

Example 1 0.2 % $Co/CeO_2$

0.75 g of [Co(III)(acac)$_3$] was dissolved in 35 $cm^3$ of analar acetone. this solution was slowly added to 60 g of $CeO_2$ with continuous stirring until a uniform paste was formed. The paste was dried over a steam bath, again with continuous stirring/kneading, until a uniform powder was obtained. The powder was stored in an oven at 150 °C overnight prior to pretreatment.

Example 2 0.3 % $Co/CeO_2$

The same method as described in Example 1 was used with 1.15 g of [Co(III)(acac)$_3$] being dissolved in 60 $cm^3$ of analar acetone.

Example 3 0.3% $Co/CeO_2$

The same method as described in Example 2 was used except that 0.8 g of [Co(II)$(acac)_2$], dissolved in 55 $cm^3$ of analar acetone, was impregnated onto 60 g of $CeO_2$.

Example 4 0.5 % $Co/CeO_2$

The same method as described in Example 3 was used with 1.35 g of [Co(II)$(acac)_2$] being dissolved in 150 $cm^3$ of analar acetone.

Example 5 0.2 % $Co/CeO_2$

[Co$(acac)_3$] (0.75 g, 2.1 mmol was dissolved in analar acetone (35 $cm^3$). The solution was slowly added to a glass dish containing $CeO_2$ (60 g), with continuous stirring until a consistent paste was formed. A further portion of acetone (10 $cm^3$) was used to ensure all the [Co$(acac)_3$] was washed onto the ceria. The paste was dried over a steam bath, with continuous stirring/kneading, until the impregnated ceria became a powder. The powder was then dried in an air oven overnight at 150 °C prior to pretreatment.

Example 6 1.4 % Co/10 % $La_2O_3/CeO_2$

$Co(NO_3)_36H_2O$ (3.5 g, 12.0 mmol) was dissolved in analar acetone (15 mmol) and added to a solution a $La(NO_3)_36H_2O$ (13.3 g, 30.7 mmol) dissolved in analar acetone (15 mmol).

The solution was slowly added to $CeO_2$ powder (50 g, ex J. M.) with careful stirring/kneading until a consistent paste was obtained. The paste was dried on the steam bath until a dry powder was obtained, which was then dried overnight in an air oven at 150 °C.

The material dried into a solid block and no pressing was necessary.

Example 7 2.1 % Co/10 % $La_2O_3/CeO_2$

This catalyst was prepared in an identical manner to the catalyst of Example 5, the amounts of materials employed being altered in accordance with the above stoichiometry.

Comparison Test 1 0.3 % $Fe/CeO_2$

Exemple 1 was repeated except that 1.12 g of [Fe(III)$(acac)_3$] was dissolved in 60 $cm^3$ of analar acetone instead of 0.75 g of [Co(III)$(acac)_3$] being dissolved in 35 $cm^3$ of analar acetone.

Comparison Test 2

Example 1 was repeared except that 0.80 g of [Ni(II)$(acac)_2$] was dissolved in 60 $cm^3$ of analar acetone instead of 0.75 g of [Co(III)$(acac)_3$] being dissolved in 35 $cm^3$ of analar acetone.

B. Catalyst pretreatment

The $Co/CeO_2$ powders obtained in Examples 1 to 4 and Comparison Tests 1 and 2 were treated individually as follows :

The powder was heated in an atmosphere of nitrogen at a rate of 0.5 °C/min to 450 °C at which temperature it was held for 6 hours before being allowed to cool again to room temperature, the whole operation being conducted in the nitrogen atmosphere. In a hydrogen atmosphere, the powder was heated at a rate of 2 °C/minute to 125 °C, maintained at this temperature for 2 hours, heated again at 2 °C/minute to 225 °C, held at this temperature for 2 hours, heated again at 2 °C/minute to 320 °C, held at this temperature for 6 hours and finally allowed to cool to room temperature, all in the hydrogen atmosphere.

The treated catalyst was then pressed to 4 tons and the resulting pellets crushed and sieved to BSS 8-20 mesh. The catalyst was then loaded into a reactor and reduced in a slow stream of hydrogen as follows : — heating at 2 °C/minute to 150 °C, then at 1 °C/minute to 225 °C and holding at this temperature for 14 hours.

The cobalt/ceria powder of Example 5 was pretreated (after pressing to 1 ton) in the manner of the following scheme :

$$R/T^{o} \xrightarrow{0.5°C/min} 450°C \xrightarrow{6.0h} 450°C \xrightarrow{10°C/min} R/T^{o} \quad (1)$$

$$\longleftarrow N2 \longrightarrow$$

$$R/T^o \xrightarrow{3°c/min} 400°C \xrightarrow{6.0h} 400°C \xrightarrow{10°C/min} R/T^o \qquad (2)$$

$$\longleftarrow H_2 \longrightarrow$$

The catalyst was pressed to 4 tons and then sieved to pass 8-20 mesh BSS.

The catalyst was then loaded into a reactor and reduced in a slow stream of hydrogen at 320 °C.

The catalysts of Examples 6 and 7 were pretreated (without pressing) in the manner of the following scheme :

$$R/T^o \xrightarrow{3°C/min} 450°C \xrightarrow{6.0h} 450°C \xrightarrow{10°C/min} R/T^o$$

$$\longleftarrow N_2 \longrightarrow$$

$$R/T^o \xrightarrow{3°C/min} 400°C \xrightarrow{6.0h} 400°C \xrightarrow{10°C/min} R/T^o$$

$$\longleftarrow H_2 \longrightarrow$$

The catalysts were sieved to 18-25 mesh BSS and loaded into the reactor.

The catalysts were reduced in the plant at 400 °C/$H_2$.

C. Catalyst testing

Examples 8 to 11 and Comparison Tests 3 and 4

At the end of the catalyst pretreatment of B above, syngas was introduced into the reactor containing in turn the pretreated catalysts of Examples 1 to 4 and Comparison Tests 1 and 2 with the pressure adjusted to 20 bar. The conditions were adjusted to those shown in the Table.

The results of testing the pretreated catalysts of Examples 1 to 4 and Comparison Tests 1 and 2 are shown as Examples 8 to 11 and Comparison Tests 3 and 4 respectively in Table 1.

Example 12

At the end of the catalyst pretreatment of B above, syngas was introduced into the reactor containing the pretreated catalyst of Example 5 under the conditions shown in Table 2.

Examples 13 and 14

At the end of the catalyst pretreatment of B above, syngas was introduced into the reactor containing in turn the pretreated catalysts of Examples 6 and 7 under the conditions shown in Table 3.

The results of testing the pretreated catalysts of Examples 6 and 7 are shown as Examples 13 and 14 in Table 3.

The results of testing the pretreated catalyst of Example 5 is shown as Example 12 in Table 2.

With reference to Table 1, the results shown for Examples 9 and 10 demonstrate that there is no significant difference in catalyst performance between a catalyst having a Co(II) precursor and one having a Co(III) precursor.

At identical low loadings (0.3 %), the supported iron and nickel catalysts are inferior in all respects to the supported cobalt catalysts.

(See Tables 1-3 page 6)

Table 1

Ceria-Supported Catalysts Prepared by Impregnation
$H_2$ : CO = 2 : 1 Molar Ratio
Pressure = 20 bar
GHSV($h^{-1}$) = 2 500

| Example | Catalyst | T/°C | %CO Converted | Carbon Molar Selectivity %* | | | | | $C_3^+$ Productivity kg/$m^3$ cat/h |
|---|---|---|---|---|---|---|---|---|---|
| | | | | $CO_2$ | $CH_4$ | $C_2$ | $C_3^+$ | $C_5^+$ | |
| 8 | Ex 1 | 282 | 41 | 6.2 | 18.3 | 4.5 | 67.9 | 49.9 | 132 |
| 9 | Ex 2 | 279 | 36 | 5.3 | 21.6 | 4.7 | 65.4 | 47.6 | 130 |
| 10 | Ex 3 | 280 | 39 | 5.6 | 21.6 | 4.6 | 65.3 | 47.3 | 135 |
| 11 | Ex 4 | 242 | 31 | 7.0 | 37.7 | 5.6 | 43.9 | 30.7 | 76 |
| Comp Test 3 | Comp Test 1 | 400 | 14 | 44.4 | 28.5 | 13.5 | 13.5 | 0 | 17 |
| Comp Test 4 | Comp Test 2 | 400 | about 3 | 61.0 | 23.5 | 8.1 | 7.7 | 0 | 2 |

* Oxygenates not included

Table 2

CO : $H_2$ ratio = 1 : 2 molar
GHSV = 2 500 $h^{-1}$
Temp = 285 °C
Pressure = 30 bar

| Ex | Catalyst | % CO conv. | Carbon Molar Selectivity | | | | | $C_3^+$ and $C_5^+$ Productivity Kgm$^3$ cat/h | |
|---|---|---|---|---|---|---|---|---|---|
| | | | $CO_2$ | $CH_4$ | $C_2$ | $C_3^+$ | $C_5^+$ | $C_3^+$ | $C_5^+$ |
| 12 | Ex. 5 | 49 | 3.38 | 17.3 | 2.8 | 73.7 | 59.2 | 188 | 151 |

Oxygenates = 2.9 % selectivity

Table 3

CO : $H_2$ ratio = 1 : 2 molar
GHSV = 2 500 $h^{-1}$
Pressure = 30 bar

| Ex | | HOS | T°C | CO % conv | Carbon Molar Selectivity % * | | | | | Productivity | |
|---|---|---|---|---|---|---|---|---|---|---|---|
| | | | | | $CO_2$ | $CH_4$ | $C_2$ | $C_3^+$ | $C_5^+$ | $C_3^+$ | $C_5^+$ |
| 13 | 6 | 188 | 275 | 51 | 21.4 | 10.8 | 4.4 | 59.3 | 47.1 | 157 | 125 |
| 14 | 7 | 70 | 270 | 55 | 18.0 | 10.6 | 4.4 | 63.8 | 52.5 | 183 | 150 |

* Oxygenates not included.

## Claims

1. A process for the production of hydrocarbons having a carbon number greater than one by contacting the synthesis gas at elevated temperature and atmospheric or superatmospheric pressure with a solid catalyst characterised in that the catalyst has a composition represented by the formula

$$Co_a \cdot A_b \cdot La_c \cdot CeO_x \qquad (I)$$

wherein

A is an alkali metal

a is greater than zero and up to 25 % w/w,

b is in the range from zero to 5 % w/w,

c is in the range from zero to 15 % w/w,

x is a number such that the valence requirements of the other elements for oxygen is satisfied, and the remainder of the composition, subject to the requirement for x, is cerium,

the percentages w/w being based on the total weight of the composition.

2. A process according to claim 1 wherein b in the formula (I) is greater than zero and the alkali metal (A) is either sodium or potassium.

3. A process according to claim 1 wherein the value of b in the formula (I) is zero.

4. A process according to any one of the preceding claims wherein the value of (a) in the formula (I) is less than 5 % w/w.

5. A process according to any one of the preceding claims wherein the value of c in the formula (I) is greater than zero.

6. A process according to any one of the preceding claims wherein the catalyst incorporates a porotectometallosilicate.

7. A process according to claim 6 wherein the porotectometallosilicate is an aluminosilicate having a silica to alumina ratio greater than 10 : 1.

8. A process according to any one of the preceding claims wherein before use the catalyst having a composition according to formula (I) is reductively activated.

9. A process according to any one of the preceding claims wherein the elevated temperature is in the range from 190 to 400 °C and the pressure is in the range from 0 to 100 bar.

10. A process according to any one of the preceding claims including a further step whereby the product, or at least a portion thereof, obtained by contacting synthesis gas with the catalyst composition of formula (I) is up-graded.

## Patentansprüche

1. Verfahren zur Herstellung von Kohlenwasserstoffen mit einer um eins größeren Kohlenstoffanzahl durch In-Berührung-Bringen von Synthesegas bei erhöhter Temperatur und atmosphärischem oder überatmosphärischem Druck mit einem festen Katalysator, dadurch gekennzeichnet, daß der Katalysator eine Zusammenseztung hat, wie sie durch die folgende Formel repräsentiert wird

$$Co_a \cdot A_b \cdot La_c \cdot CeO_x \qquad (I)$$

in welcher

A ein Alkalimetall ist

a größer als Null und bis zu 25 Gew.-% ist,

b im Bereich von Null bis 5 Gew.-% liegt,

c im Bereich von Null bis 15 Gew.-% liegt,

x eine solche Zahl ist, daß die Valenzerfordernisse der anderen Elemente für Sauerstoff erfülltwerden, und der Rest der Zusammensetzung, in Abhängigkeit von dem Erfordernis für x, Cerium ist,

wobei die Gewichtsprozentangaben auf dem Gesamtgewicht der Zusammensetzung beruhen.

2. Verfahren nach Anspruch 1, worin b in Formel (I) größer als Null und das Alkalimetall (A) entweder Natrium oder Kalium ist.

3. Verfahren nach Anspruch 1, worin der Wert von b in Formel (I) Null ist.

4. Verfahren nach einem der vorgehenden Ansprüche, worin der Wert von (a) in Formel (I) weniger als 5 Gew.-% ist.

5. Verfahren nach einem der vorgehenden Ansprüche, worin der Wert von c in Formel (I) größer als Null ist.

6. Verfahren nach einem der vorgehenden Ansprüche, worin der Katalysator ein Porotectometallosilikat enthält.

7. Verfahren nach Anspruch 6, worin das Porotectometallosilikat ein Aluminosilikat ist mit einem Kieselsäure zu Aluminiumoxid-Verhältnis von größer als 10 : 1.

8. Verfahren nach einem der vorgehenden Ansprüche, worin der Katalysator, der vor seiner Verwendung die Zusammensetzung gemäß Formel (I) hat, reduktiv aktiviert wird.

9. Verfahren nach einem der vorgehenden Ansprüche, worin die erhöhte Temperatur im Bereich von 190 bis 400 °C und der Druck im Bereich von 0 bis 100 bar liegen.

10. Verfahren nach einem der vorgehenden Ansprüche, welches einen weiteren Schritt einschließt, bei welchem das Produkt, oder zumindest ein Teil davon, welches erhalten wird durch In-Berührung-Bringen des Synthesegases mit der Katalysatorzusammensetzung der Formel (I) aufbereitet wird.

**Revendications**

1. Procédé pour la production d'hydrocarbures ayant un nombre d'atomes de carbone supérieur à un, par mise du gaz de synthèse en contact, à température élevée et à une pression atmosphérique ou supérieure à la pression atmosphérique, avec un catalyseur solide, procédé caractérisé en ce que le catalyseur possède une composition représentée par la formule

$$Co_a \cdot A_b \cdot La_c \cdot CeO_x \qquad (I)$$

dans laquelle

A représente un métal alcalin

a est supérieur à zéro et peut valoir jusqu'à 25 % en poids/poids,

b se situe entre zéro et 5 % en poids/poids,

c se situe entre zéro et 15 % en poids/poids,

x est un nombre tel que les exigences de valence des autres éléments soient satisfaites à l'égard de l'oxygène, et le reste de la composition, en tenant compte de l'exigence spécifiée pour x, est constitué par du cérium,

les pourcentages en poids/poids étant basés sur le poids total de la composition.

2. Procédé selon la revendication 1, dans lequel b, dans la formule (I), est supérieur à zéro, et le métal alcalin (A) est le sodium ou le potassium.

3. Procédé selon la revendication 1, dans lequel la valeur de (b) dans la formule (I) est nulle.

4. Procédé selon l'une quelconque des revendications précédentes, dans lequel la valeur de (a) dans la formule (I) est inférieure à 5 % en poids/poids.

5. Procédé selon l'une quelconque des revendications précédentes, dans lequel la valeur de (c) dans la formule (I) est supérieure à zéro.

6. Procédé selon l'une quelconque des revendications précédentes, dans lequel le catalyseur incorpore un porotectométallosilicate.

7. Procédé selon la revendication 6, dans lequel le porotectométallosilicate est un aluminosilicate présentant un rapport de la silice à l'alumine supérieur à 10 : 1.

8. Procédé selon l'une quelconque des revendications précédentes, dans lequel, avant son utilisation, le catalyseur ayant une composition selon la formule (I) est soumis à activation réductrice.

9. Procédé selon l'une quelconque des revendications précédentes, dans lequel la température élevée se situe entre 190 et 400 °C, et la pression se situe entre 0 et 100 bars.

10. Procédé selon l'une quelconque des revendications précédentes, comprenant une étape supplémentaire au cours de laquelle le produit, ou au moins une partie de ce produit, que l'on obtient en mettant du gaz de synthèse en contact avec la composition de catalyseur de formule (I), est amélioré par valorisation.